(19) Europäisches Patentamt
European Patent Office
Office européen des brevets

(11) EP 1 974 772 A1

(12) # DEMANDE DE BREVET EUROPEEN

(43) Date de publication:
**01.10.2008 Bulletin 2008/40**

(21) Numéro de dépôt: **07123272.2**

(22) Date de dépôt: **14.12.2007**

(51) Int Cl.:
***A61Q 5/06*** *(2006.01)* ***A61K 8/895*** *(2006.01)*
***A61K 8/89*** *(2006.01)*

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **20.12.2006 FR 0655755**

(71) Demandeur: **L'ORÉAL**
**75008 Paris (FR)**

(72) Inventeur: **Benabdillah, Katarina**
**95130 Le Plessis-Bouchard (FR)**

(74) Mandataire: **Bourdeau, Françoise**
**L'OREAL - D.I.P.I.**
**25-29 Quai Aulagnier**
**92665 Asnières-sur-Seine Cedex (FR)**

(54) **Obtention de coiffures structurees mettant en oeuvre une composition comprenant des composes silicones reactifs**

(57) La présente invention a pour le traitement de fibres kératiniques humaines pour l'obtention de coiffures structurées, par exemple méchées, consistant à appliquer une composition comprenant au moins un composé X et Y, dont l'un au moins est un composé siliconé, ces composés étant de plus susceptibles de réagir ensemble par une réaction d'hydrosilylation, ou par une réaction de condensation ou par une réaction de réticulation en présence d'un peroxyde.

EP 1 974 772 A1

**Description**

La présente invention a pour objet le traitement de fibres kératiniques humaines pour l'obtention de coiffures structurées, consistant à appliquer une composition comprenant au moins un composé X et Y, dont l'un au moins est un composé siliconé, ces composés étant de plus susceptibles de réagir ensemble par une réaction d'hydrosilylation, de condensation ou de réticulation.

La préparation de certaines coiffures sophistiquées constituées de nattes, de pics, de boucles, de chignons, etc., est bien souvent longue et complexe et nécessite dans certains cas l'intervention d'un coiffeur. C'est pourquoi il est souhaité de conserver intacte une telle coiffure pendant quelques jours, voire plus longtemps.

L'inconvénient est que les produits de coiffage employés pour l'obtention de telles coiffures s'éliminent au premier shampooing et ne permettent pas de maintenir la coiffure le temps souhaité.

Afin de remédier à cet inconvénient, il est envisageable d'accroître la rémanence du produit en effectuant directement une polymérisation de certains monomères au niveau des cheveux. Ainsi, le document US 4 344 763 décrit une composition de fixation des cheveux à partir d'une silicone réactive aminoalkylalcoxysilane et un titanate d'ester. Il est aussi connu d'effectuer des gainages des cheveux à partir d'une composition comprenant un monomère électrophile de type cyanoacrylate, notamment dans les demandes de brevet FR 2 833 489. Une telle composition permet d'obtenir des cheveux parfaitement gainés et non gras. Cependant, le gainage obtenu ne donne pas une totale satisfaction face aux agents extérieurs tels que le lavage et la transpiration. Par ailleurs le gainage obtenu est sensible aux corps gras tels que le sébum.

Le but de la présente invention est de développer un nouveau procédé de traitement des cheveux qui permet d'obtenir une fixation durable tout en conservant de bonnes propriétés cosmétiques.

La présente invention a donc pour objet un procédé de traitement de fibres kératiniques humaines, telles que les cheveux, pour l'obtention de coiffures structurées, dans lequel on applique une composition comprenant au moins un composé X, au moins un composé Y, au moins un des composés X ou Y étant siliconé, lesdits composés X et Y étant susceptibles de réagir ensemble par une réaction d'hydrosilylation, ou par une réaction de condensation ou par une réaction de réticulation en présence d'un peroxyde.

Le procédé selon l'invention permet notamment d'obtenir des mèches comprenant un ensemble d'au moins 10, plus avantageusement d'au moins 50 fibres, collées entre elles. De préférence, la longueur minimale des fibres est de 10 cm.

Le procédé peut aussi être avantageusement mis en oeuvre pour l'obtention de coiffures résistantes aux shampooings sur des cheveux africains frisés et défrisés.

Par ailleurs, on a constaté que le gainage obtenu est homogène, lisse et possède une excellente adhésion sur les cheveux. En outre, ce gainage est rémanent à plusieurs shampooings, ce qui permet de conserver la coiffure plus longtemps.

Mais d'autres caractéristiques et avantage de la présente invention apparaîtront plus clairement à la lecture de la description et des exemples qui vont suivre.

Il est à noter que, dans ce qui suit, les bornes encadrant un domaine de valeurs sont comprises dans ce domaine à moins qu'une indication contraire ne soit prévue.

Comme cela a été indiqué auparavant, la composition appliquée sur les fibres comprend au moins un composé X, au moins un composé Y, dont l'un au moins est un composé siliconé.

**Composés X et Y**

Par composé siliconé, on entend un composé comprenant au moins deux unités organosiloxanes. Selon un mode de réalisation particulier, les composés X et les composés Y sont siliconés. Les composés X et Y peuvent être aminés ou non aminés. Ils peuvent comprendre des groupes polaires pouvant être choisis parmi les groupes suivants -COOH, $-COO^-$, -COO- , -OH , $-NH_2$ , -NH-,-NR-, $-SO_3H$, $-SO_3^-$, $-OCH_2CH_2-$, $-O-CH_2CH_2CH_2-$, $-O-CH_2CH(CH_3)-$, $-NR_3^+$, -SH, $-NO_2$, I, Cl, Br, -CN, $-PO_4{}^{3-}$, -CONH- , -CONR-, $-CONH_2$, -CSNH-, $-SO_2-$ , -SO-, $-SO_2NH-$, -NHCO- , $-NHSO_2-$ , -NHCOO-, -OCONH-, -NHCSO- et -OCSNH-, R représentant un groupe alkyle.

Selon un autre mode de réalisation, au moins un des composés X et Y est un polymère dont la chaîne principale est formée majoritairement d'unités organosiloxanes.

Parmi les composés siliconés cités ci-après, certains peuvent présenter à la fois des propriétés filmogènes et adhésives, selon par exemple leur proportion en silicone ou selon qu'on les utilise en mélange avec un additif particulier. Il est par conséquent possible de moduler les propriétés filmogènes ou les propriétés adhésives de tels composés selon l'utilisation envisagée, c'est en particulier le cas pour les silicones élastomères réactives dites " room temperature vulcanization" .

Les composés X et Y peuvent réagir ensemble à une température variant entre la température ambiante et 180°C. Avantageusement les composés X et Y sont susceptibles de réagir ensemble à température ambiante (20 ±

5°C) et pression atmosphérique, avantageusement en présence d'un catalyseur, par une réaction d'hydrosilylation ou une réaction de condensation, ou une réaction de réticulation en présence d'un peroxyde.

**1- Composés X et Y susceptibles de réagir par hydrosilylation**

Selon un mode de réalisation, les composés X et Y sont susceptibles de réagir par hydrosilylation, cette réaction pouvant être de manière simplifiée schématisée comme suit :

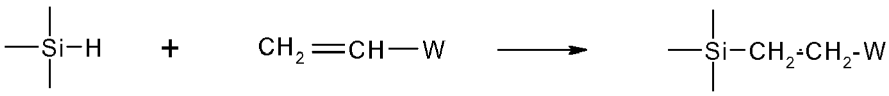

avec W représentant une chaîne carbonée et/ou siliconée contenant un ou plusieurs groupements aliphatiques insaturés.

Dans ce cas, le composé X peut être choisi parmi les composés siliconés comprenant au moins deux groupements aliphatiques insaturés. A titre d'exemple, le composé X peut comprendre une chaîne principale siliconée dont les groupements aliphatiques insaturés sont pendants à la chaîne principale (groupe latéral) ou situés aux extrémités de la chaîne principale du composé (groupe terminal). On appellera, dans la suite de la description, ces composés particuliers des polyorganosiloxanes à groupements aliphatiques insaturés.

Dans ce cas, le composé X peut être choisi parmi les composés siliconés comprenant au moins deux groupements aliphatiques insaturés.

A titre d'exemple, le composé X peut comprendre une chaîne principale siliconée dont les groupements aliphatiques insaturés sont pendants à la chaîne principale (groupe latéral) ou situés aux extrémités de la chaîne principale du composé (groupe terminal). On appellera, dans la suite de la description, ces composés particuliers des polyorganosiloxanes à groupements aliphatiques insaturés.

Selon un mode de réalisation, le composé X est choisi parmi les polyorganosiloxanes comprenant au moins deux groupements aliphatique insaturés, par exemple deux ou trois groupements vinyliques ou allyliques, liés chacun à un atome de silicium.

Selon un mode de réalisation avantageux, le composé X est choisi parmi les polyorganosiloxanes comprenant des unités siloxanes de formule :

$$R_mR'SiO_{\frac{(3-m)}{2}} \qquad (I)$$

dans laquelle :

- R représente un groupe hydrocarboné monovalent, linéaire ou cyclique, comprenant de 1 à 30 atomes de carbone, de préférence de 1 à 20, et mieux de 1 à 10 atomes de carbone, comme par exemple un radical alkyle à chaîne courte, comprenant par exemple de 1 à 10 atomes de carbone, en particulier un radical méthyle ou encore un groupement phényle, de préférence un radical méthyle,
- m est égal à 1 ou 2 et
- R' représente :

  o un groupement hydrocarboné aliphatique insaturé comprenant de 2 à 10, de préférence de 2 à 5 atomes de carbone comme par exemple un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène ; on peut citer comme groupement R' les groupements vinyle, allyle et leurs mélanges ; ou
  o un groupement hydrocarboné cyclique insaturé comprenant de 5 à 8 atomes de carbone comme par exemple un groupe cyclohexenyle.

De préférence R' est un groupement hydrocarboné aliphatique insaturé, de préférence un groupe vinyle.

Selon un mode de réalisation particulier, le polyorganosiloxane comprend également des unités de formule

$$R_nSiO_{\frac{(4-n)}{2}} \quad (II)$$

dans laquelle R est un groupe tel que défini plus haut, et n est égal à 1, 2 ou 3.

Selon une variante, le composé X peut être une résine de silicone comprenant au moins deux insaturations éthyléniques, ladite résine étant apte à réagir avec le composé Y par hydrosilylation. On peut citer par exemple les résines de type MQ ou MT portant elle-même des extrémités réactives insaturées $-CH=CH_2$.

Ces résines sont des polymères d'organosiloxanes réticulés.

La nomenclature des résines de silicone est connue sous le nom de "MDTQ", la résine étant décrite en fonction des différentes unités monomériques siloxane qu'elle comprend, chacune des lettres "MDTQ" caractérisant un type d'unité.

La lettre M représente l'unité monofonctionelle de formule $(CH_3)_3SiO_{1/2}$, l'atome de silicium étant relié à un seul atome d'oxygène dans le polymère comprenant cette unité.

La lettre D signifie une unité difonctionnelle $(CH_3)_2SiO_{2/2}$ dans laquelle l'atome de silicium est relié à deux atomes d'oxygène

La lettre T représente une unité trifonctionnelle de formule $(CH_3)SiO_{3/2}$.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényle ou bien encore un groupe hydroxyle.

Enfin, la lettre Q signifie une unité tétrafonctionnelle $SiO_{4/2}$ dans laquelle l'atome de silicium est lié à quatre atomes d'hydrogène eux mêmes liés au reste du polymère. Comme exemples de telles résine, on peut citer les résines de silicone MT telles que les poly(phényl-vinylsilsesquioxane) comme celle commercialisées sous la référence SST-3PV1 par la société Gelest.

De préférence, les composés X comprennent de 0,01 à 1 % en poids de groupes aliphatiques insaturés.

Avantageusement, le composé X est choisi parmi les polyorganopolysiloxanes, notamment ceux comprenant les unités siloxanes (I) et éventuellement (II) décrites précédemment.

Le composé Y comprend de préférence au moins deux groupes Si-H (groupes hydrogénosilanes) libres.

Le composé Y peut être avantageusement choisi parmi les organosiloxanes comprenant au moins une unité alkylhydrogénosiloxane de formule suivante :

$$R_pHSiO_{\frac{(3-p)}{2}} \quad (III)$$

dans laquelle :

> R représente un groupe hydrocarboné monovalent, linéaire ou cyclique, comprenant de 1 à 30 atomes de carbone, comme par exemple un radical alkyle ayant de 1 à 30 atomes de carbone, de préférence de 1 à 20 et mieux de 1 à 10 atomes de carbone, en particulier un radical méthyle, ou encore un groupement phényle et p est égal à 1 ou 2. De préférence R est un groupement hydrocarboné, de préférence le méthyle.

Ces composés Y organosiloxanes à unités alkylhydrogénosiloxanes peuvent comprendre en outre des unités de formule :

$$R_nSiO_{\frac{(4-n)}{2}} \quad (II)$$

telles que définies plus haut.

Le composé Y peut être une résine de silicone comprenant au moins un motif choisis parmi les motifs M, D, T, Q tels que définis ci-dessus et comprenant au moins un groupe Si-H telles que les poly(méthyl-hydridosilsesquioxane)

commercialisées sous la référence SST-3MH1.1 par la société Gelest

De préférence, ces composés Y organosiloxanes comprennent de 0,5 à 2,5% en poids de groupes Si-H.

Avantageusement, les radicaux R représentent un groupement méthyle dans les formules (I), (II), (III) ci-dessus.

De préférence, ces organosiloxanes Y comprennent des groupes terminaux de formule $(CH_3)_3SiO_{1/2}$.

Avantageusement, les organosiloxanes Y comprennent au moins deux unités alkylhydrogénosiloxane de formule $(H_3C)(H)SiO$ et comprennent éventuellement des unités $(H_3C)_2SiO$.

De tels composés Y organosiloxanes à groupements hydrogénosilane sont décrits par exemple dans le document EP 0465744.

Selon une variante, le composé X est choisi parmi les oligomères ou polymères organiques (par organique, on entend des composés dont la chaîne principale est non siliconée, de préférence des composés ne comprenant pas d'atomes de silicium) ou parmi les polymères ou oligomères hybrides organique/silicone, lesdits oligomères ou polymères portant au moins 2 groupements aliphatiques insaturés réactifs, le composé Y étant choisi parmi les hydrogénosiloxanes cités ci-dessus.

Le composé X, de nature organique, peut alors être choisi parmi les polymères ou oligomères vinyliques, (méth)acryliques, les polyesters, les polyuréthanes et/ou les polyurées, les polyéthers, les perfluoropolyéthers, les polyoléfines telles que le polybutène, le polyisobutylène, les dendrimères ou les polymères hyper-ramifiés organiques, ou leurs mélanges.

En particulier, le polymère organique ou la partie organique du polymère hybride peut être choisi parmi les polymères suivants :

a) Les polyesters à insaturation(s) éthylénique(s) :

Il s'agit d'un groupe de polymères de type polyester présentant au moins 2 doubles liaisons éthyléniques, réparties de manière aléatoire dans la chaîne principale du polymère. Ces polyesters insaturés sont obtenus par polycondensation d'un mélange :

- de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturations éthyléniques tels que les dimères des acides oléique ou linoléique décrits dans la demande EP-A-959 066 (paragraphe [0021]) commercialisés sous les dénominations Pripol® par la société Unichema ou Empol® par la société Henkel, tous ces diacides devant être exempts de doubles liaisons éthyléniques polymérisables,
- de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le le bisphénol A et le bisphénol B, et/ou de dimères diols issus de la réduction des dimères d'acides gras tels que définis précédemment, et
- d'un ou de plusieurs diacides carboxyliques ou leurs anhydrides comportant au moins une double liaison éthylénique polymérisable et ayant de 3 à 50 atomes de carbone, de préférence de 3 à 20 et mieux de 3 à 10 atomes de carbone, tels que l'acide maléique, l'acide fumarique ou l'acide itaconique.

b) les polyesters à groupes (méth)acrylate latéraux et/ou terminaux :

Il s'agit d'un groupe de polymères de type polyester obtenus par polycondensation d'un mélange :

- de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturation éthyléniques tels que les dimères des acides oléique ou linoléique décrits dans la demande EP-A-959 066 (paragraphe [0021]) commercialisés sous les dénominations Pripol® par la société Unichema ou Empol® par la société Henkel, tous ces diacides devant être exempts de doubles liaisons éthyléniques polymérisables,

- de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le bisphénol A et le bisphénol B, et
- d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.

Ces polyesters diffèrent de ceux décrits ci-dessus sous le point a) par le fait que les doubles liaisons éthyléniques ne sont pas situées dans la chaîne principale mais sur des groupes latéraux ou à l'extrémité des chaînes. Ces doubles liaisons éthyléniques sont celles des groupes (méth)acrylate présents dans le polymère.
De tels polyesters sont commercialisés par exemple par la société UCB sous les dénominations EBECRYL® (EBECRYL® 450 : masse molaire 1600, en moyenne 6 fonctions acrylate par molécule, EBECRYL® 652 : masse molaire 1500, en moyenne 6 fonctions acrylate par molécule, EBECRYL® 800 : masse molaire 780, en moyenne 4 fonctions acrylate par molécule, EBECRYL® 810 : masse molaire 1000, en moyenne 4 fonctions acrylate par molécule, EBECRYL® 50 000 : masse molaire 1500, en moyenne 6 fonctions acrylate par molécule).

c) les polyuréthannes et/ou polyurées à groupes (méth)acrylate, obtenus par polycondensation :

- de diisocyanates, triisocyanates et/ou polyisocyanates aliphatiques cycloaliphatiques et/ou aromatiques ayant notamment de 4 à 50, de préférence de 4 à 30 atomes de carbone, tels que l'hexaméthylènediisocyanate, l'isophoronediisocyanate, le toluènediisocyanate, le diphénylméthanediisocyanate ou les isocyanurates de formule :

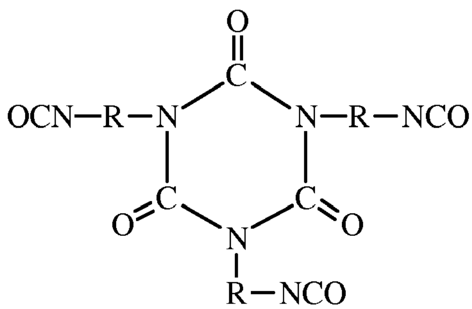

résultant de la trimérisation de 3 molécules de diisocyanates OCN-R-CNO, où R est un radical hydrocarboné linéaire, ramifié ou cyclique comportant de 2 à 30 atomes de carbone ;
- de polyols, notamment de diols, exempts d'insaturations éthyléniques polymérisables, tels que le 1,4-butanediol, l'éthylèneglycol ou le triméthylolpropane, et/ou de polyamines, notamment de diamines, aliphatiques, cycloaliphatiques et/ou aromatiques ayant notamment de 3 à 50 atomes de carbone, telles que l'éthylènediamine ou l'hexaméthylènediamine, et
- d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
De tels polyuréthannes/polyurées à groupes acrylates sont commercialisés par exemple sous la dénomination SR 368 (tris(2-hydroxyéthyl)isocyanurate-triacrylate) ou CRAYNOR® 435 par la société CRAY VALLEY, ou sous la dénomination EBECRYL® par la société UCB (EBECRYL® 210 : masse molaire 1500, 2 fonctions acrylate par molécule, EBECRYL® 230 : masse molaire 5000, 2 fonctions acrylate par molécule, EBECRYL® 270 : masse molaire 1500, 2 fonctions acrylate par molécule, EBECRYL® 8402 : masse molaire 1000, 2 fonctions acrylate par molécule, EBECRYL® 8804 : masse molaire 1300, 2 fonctions acrylate par molécule, EBECRYL® 220 : masse molaire 1000, 6 fonctions acrylate par molécule, EBECRYL® 2220 : masse molaire 1200, 6 fonctions acrylate par molécule, EBECRYL® 1290 : masse molaire 1000, 6 fonctions acrylate par molécule, EBECRYL® 800 : masse molaire 800, 6 fonctions acrylate par molécule).
On peut également citer les polyuréthannes aliphatiques diacrylate hydrosolubles commercialisés sous les dénominations EBECRYL® 2000, EBECRYL® 2001 et EBECRYL® 2002, et les polyuréthannes diacrylate en dispersion aqueuse commercialisés sous les dénominations commerciales IRR® 390, IRR® 400, IRR® 422

IRR® 424 par la société UCB.

d) les polyéthers à groupes (méth)acrylate obtenus par estérification, par l'acide (méth)acrylique, des groupes hydroxyle terminaux d'homopolymères ou de copolymères d'alkylèneglycols en $C_{1-4}$, tels que le polyéthylèneglycol, le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé. Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL® 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL® 160 par la société UCB. Des triacrylates de triméthylolpropane polypropoxylé sont commercialisés par exemple sous les dénominations SR 492 et SR 501 par la société CRAY VALLEY.

e) les époxyacrylates obtenus par réaction entre

- au moins un diépoxyde choisi par exemple parmi :

  (i) l'éther diglycidylique de bisphénol A
  (ii) une résine diépoxy résultant de la réaction entre l'éther diglycidylique de bisphénol A et l'épichlorhydrine,
  (iii) une résine époxyester à extrémités $\alpha,\omega$-diépoxy résultant de la condensation d'un diacide carboxylique ayant de 3 à 50 atomes de carbone avec un excès stoechiométrique de (i) et/ou (ii),
  (iv) une résine époxyéther à extrémités $\alpha,\omega$-diépoxy résultant de la condensation d'un diol ayant de 3 à 50 atomes de carbone avec un excès stoechiométrique de (i) et/ou (ii),
  (v) les huiles naturelles ou synthétiques portant au moins 2 groupes époxyde, telles que l'huile de soja époxydée, l'huile de lin époxydée et l'huile de vernonia époxydée,
  (vi) un polycondensat phénol-formaldéhyde (résine Novolac®), dont les extrémités et/ou les groupes latéraux ont été époxydés, et

- un ou plusieurs acides carboxyliques ou polyacides carboxyliques comportant au moins une double liaison éthylénique en $\alpha,\beta$ du groupe carboxylique comme l'acide (méth)acrylique ou l'acide crotonique ou les esters d'acide (méth)acrylique et d'un diol ou polyol ayant de 2 à 20 atomes de carbone, de préférence de 2 à 6 atomes de carbone tels que le (méth)acrylate de 2-hydroxyéthyle.
  De tels polymères sont commercialisés par exemple sous les dénominations SR 349, SR 601, CD 541, SR 602, SR 9036, SR 348, CD 540, SR 480, CD 9038 par le société CRAY VALLEY, sous les dénominations EBECRYL® 600 et EBECRYL® 609, EBECRYL® 150, EBECRYL® 860, EBECRYL® 3702 par la société UCB et sous les dénominations PHOTOMER® 3005 et PHOTOMER® 3082 par la société HENKEL.

f) les poly(méth)acrylates d'(alkyle en $C_{1-50}$), ledit alkyle étant linéaire, ramifié ou cyclique, comportant au moins deux fonctions à double liaison éthylénique portées par les chaînes hydrocarbonées latérales et/ou terminales.
De tels copolymères sont commercialisés par exemple sous les dénominations IRR® 375, OTA® 480 et EBECRYL®2047 par la société UCB.

g) les polyoléfines telles que le polybutène, le polyisobutylène.

h) les perfluoropolyéthers à groupes acrylate obtenus par estérification, par exemple par l'acide (méth)acrylique, de perfluoropolyéthers portant des groupes hydroxyle latéraux et/ou terminaux.
De tels perfluoropolyéthers $\alpha,\omega$-diols sont décrits notamment dans EP-A-1057849 et sont commercialisés par la société AUSIMONT sous la dénomination FOMBLIN® Z DIOL.

i) les dendrimères et polymères hyperramifiés portant des groupes terminaux (méth)acrylate ou (méth)acrylamide obtenus respectivement par estérification ou amidification de dendrimères et de polymères hyperramifiés à fonctions terminales hydroxyle ou amino, par de l'acide (méth)acrylique.
Les dendrimères (du grec dendron = arbre) sont des molécules polymères "arborescentes", c'est-à-dire très ramifiées inventées par D. A. Tomalia et son équipe au début des années 90 (Donald A. Tomalia et al., Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST® par la société DENDRITECH.
Les polymères hyperramifiés sont des polycondensats, généralement de type polyester, polyamide ou polyéthylèneamine, obtenus à partir de monomères multifonctionnels, qui ont une structure arborescente similaire à celle des dendrimères mais beaucoup moins régulière que celle-ci (voir par exemple WO-A-93/17060 et WO 96/12754). La société PERSTORP commercialise sous la dénomination BOLTORN® des polyesters hyperramifiés. On trouvera sous la dénomination COMBURST® de la société DENDRITECH des polyéthylèneamines hyperramifiées. Des poly (esteramide) hyperramifiés à extrémités hydroxyle sont commercialisés par la société DSM sous la dénomination HYBRANE®.
Ces dendrimères et polymères hyperramifiés estérifiés ou amidifiés par l'acide acrylique et/ou méthacrylique se distinguent des polymères décrits sous les points a) à h) ci-dessus par le très grand nombre de doubles liaisons éthyléniques présentes. Cette fonctionnalité élevée, le plus souvent supérieure à 5, les rend particulièrement utiles en leur permettant de jouer un rôle de "noeud de réticulation", c'est-à-dire de site de réticulation multiple.
On peut donc utiliser ces polymères dendritiques et hyperramifiés en association avec un ou plusieurs des polymères et/ou oligomères a) à h) ci-dessus.

**1a Composés réactifs additionnels**

Selon un mode de réalisation, les compositions comprenant le composé X et/ou Y peuvent comprendre en outre au moins un composé réactif additionnel tel que :

- les particules organiques ou minérales comprenant à leur surface au moins 2 groupements aliphatiques insaturés, on peut citer par exemple les silices traitées en surface par exemple par des composés siliconés à groupements vinyliques tels que par exemple la silice traitée cyclotétraméthyl tétravinylsiloxane,
- des composés silazanes tels que l'hexaméthyldisilazane.

**1b Catalyseur**

La réaction d'hydrosilylation se fait avantageusement en présence d'un catalyseur qui peut être présent dans l'une ou l'autre des compositions comprenant le composé X et/ou le composé Y ou dans une composition séparée, le catalyseur étant de préférence à base de platine ou d'étain.
On peut citer par exemple les catalyseurs à base de platine déposé sur un support de gel de silice ou de poudre de charcoal (charbon), le chlorure de platine, les sels de platine et d'acides chloroplatiniques.
On utilise de préférence les acides chloroplatiniques sous forme hexahydrate ou anhydre, facilement dispersible dans les milieux organosiliconés.
On peut également citer les complexes de platine tels que ceux à base d'acide chloroplatinique hexahydrate et de divinyl tetramethyldisiloxane
La catalyseur peut être présent dans l'une ou l'autre des compositions utiles dans la présente invention en une teneur allant 0,0001% à 20% en poids par rapport au poids total de la composition le comprenant.
On peut également introduire dans les compositions de l'invention des inhibiteurs ou retardateurs de polymérisation, et plus particulièrement des inhibiteurs du catalyseur, ceci afin d'accroître la stabilité de la composition dans le temps ou de retarder la polymérisation. De façon non limitative, on peut citer les polyméthylvinylsiloxanes cycliques, et en particulier le tétravinyl tétraméthyl cyclotétrasiloxane, les alcools acétyléniques, de préférence volatiles, tels que le méthylisobutynol.
La présence de sels ioniques, tels que l'acétate de sodium, dans l'une et/ou l'autre des première et seconde compositions peut avoir une influence dans la vitesse de polymérisation des composés.
A titre d'exemple d'une combinaison de composés X et Y réagissant par hydrosilylation, on peut citer les références suivantes proposée par la société Dow Corning : DC 7-9800 Soft Skin Adhesive Parts A & B, ainsi que les mélanges A et B suivants préparés par Dow Corning :

**Mélange A :**

| **Ingrédient (Nom INCI)** | **N°CAS** | **Teneurs (pds%)** | **Fonction** |
|---|---|---|---|
| Dimethyl Siloxane, Dimethylvinylsiloxy-terminated | 68083-19-2 | 55-95 | Polymère |

(suite)

| Ingrédient (Nom INCI) | N°CAS | Teneurs (pds%) | Fonction |
|---|---|---|---|
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

**Mélange B :**

| Ingrédient (Nom INCI) | N°CAS | Teneurs (pds%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| Dimethyl, Methylhydrogen Siloxane, trimethylsiloxy-terminated | 68037-59-2 | 1-10 | Polymère |

De façon avantageuse, les composés X et Y sont choisis parmi les composés siliconés susceptibles de réagir par hydrosilylation ; en particulier le composé X est choisi parmi les polyorganosiloxanes comprenant des unités de formule (I) décrits ci-dessus et le composé Y est choisi parmi les organosiloxanes comprenant des unités alkylhydrogénosiloxanes de formule (III) décrits ci-dessus. Selon un mode de réalisation particulier, le composé X est un polydiméthylsiloxane à groupements vinyliques terminaux, et le composé Y est le méthylhydrogénosiloxane.

**<u>2/ Composés X et Y susceptibles de réagir par condensation</u>**

Selon ce mode de réalisation, les composés X et Y sont susceptibles de réagir par condensation, soit en présence d'eau (hydrolyse) par réaction de 2 composés porteurs de groupements alcoxysilanes, soit par condensation dite « directe » par réaction d'un composé porteur de groupement(s) alcoxysilane(s) et d'un composé porteur de groupement(s) silanol(s) ou par réaction de 2 composés porteurs de groupement(s) silanol(s).

Lorsque la condensation se fait en présence d'eau, en particulier l'eau apportée par une source extérieure, par exemple par humidification préalable des cheveux (par exemple par un brumisateur).

Dans ce mode de réaction par condensation, les composés X et Y, identiques ou différents, peuvent donc être choisis parmi les composés siliconés dont la chaîne principale comprend au moins deux groupes alcoxysilane et/ou au moins deux groupes silanol (Si-OH), latéraux et/ou en bout de chaîne.

Selon un mode de réalisation avantageux, les composés X et/ou Y sont choisis parmi les polyorganosiloxanes comprenant au moins deux groupes alcoxysilane. Par groupe « alcoxysilane », on entend un groupe comprenant au moins une partie -Si-OR, R étant un groupe alkyle comprenant de 1 à 6 atomes de carbone.

Les composés X et Y sont notamment choisis parmi les polyorganosiloxanes comprenant des groupes terminaux alcoxysilanes, plus spécifiquement ceux qui comprennent au moins 2 groupes alcoxysilanes terminaux, de préférence trialcoxysilanes terminaux.

Ces composés X et/ou Y comprennent de préférence de façon majoritaire des unités de formule

$$R^9{}_8SiO_{(4-s)/2}, \qquad (IV)$$

dans laquelle $R^9$ représente indépendamment un radical choisi parmi les groupes alkyles comprenant de 1 à 6 atomes de carbone, le phényle, les groupes alkyl fluorés, et s est égal à 0, 1, 2 ou 3. De préférence, $R^9$ représente indépendamment un groupe alkyle comprenant de 1 à 6 atomes de carbone. Comme groupe alkyle, on peut citer notamment le méthyle, le propyle, le butyle, l'hexyle et leurs mélanges, de préférence le méthyle ou l'éthyle. Comme groupe fluoroalkyle, on peut citer le 3, 3, 3-trifluoropropyle.

Selon un mode de réalisation particulier, les composés X et Y, identiques ou différents, sont des polyorganosiloxanes comprenant des unités de formule

$$(R^9{}_2SiO_2)_f - \qquad (V)$$

dans laquelle $R^9$ est tel que décrit ci-dessus, de préférence $R^9$ est un radical méthyle, et f est notamment tel que le polymère présente avantageusement une viscosité à 25°C allant de 0,5 à 3000 Pa.s, de préférence allant de 5 à 150 Pa.s et/ou f est notamment un nombre allant de 2 à 5000, de préférence de 3 à 3000, mieux encore de 5 à 1000.

Ces composés X et Y polyorganosiloxanes comprennent au moins deux groupes trialcoxysilanes terminaux par molécule de polymère, lesdits groupes ayant la formule suivante

$$-ZSiR^1_x(OR)_{3-x-} \qquad (VI)$$

dans laquelle
les radicaux R représentent indépendamment un groupe méthyle, éthyle, n-propyle, isopropyle, n-butyle, sec-butyle, isobutyle, de préférence un groupe méthyle ou éthyle,
$R^1$ est un groupe méthyle ou éthyle,
x est égal à 0 ou 1, de préférence x est égal à 0 et
Z est choisi parmi : les groupes hydrocarbonés divalents ne comportant pas d'insaturation éthylénique et comprenant de 1 à 18 atomes de carbone (groupes alkylène), les combinaisons de radicaux hydrocarbonés divalents et de segments siloxanes de formule (IX) suivante :

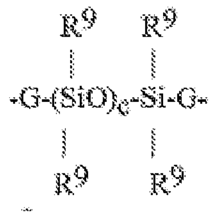

(IX)

$R^9$ étant tel que décrit plus haut, G est un radical hydrocarboné divalent ne comportant pas d'insaturation éthylénique et comprenant de 1 à 18 atomes de carbone et c est un entier allant de 1 à 6.
Z et G peuvent être notamment choisis parmi les groupements alkylènes tels que le méthylène, l'éthylène, le propylène, le butylène, le pentylène, l'hexylène, les groupements arylène tels que le phenylène.

De préférence, Z est un groupe alkylène, et mieux ethylène.

Ces polymères peuvent présenter en moyenne au moins 1,2 groupements terminaux ou chaînes terminales trialcoxysilanes par molécule, et de préférence en moyenne au moins 1,5 groupements terminaux trialcoxysilanes par molécule. Ces polymères pouvant présenter au moins 1,2 groupements terminaux trialcoxysilanes par molécule, certains peuvent comprendre d'autre types de groupes terminaux tels que des groupements terminaux de formule $CH_2=CH-SiR^9_2-$ ou de formule $R^6_3-Si-$, dans laquelle $R^9$ est tel que défini plus haut et chaque groupe $R^6$ est indépendamment choisi parmi les groupes $R^9$ ou vinyle. On peut citer comme exemples de tels groupements terminaux les groupes triméthoxysilane, triéthoxysilane, vinyldiméthoxysilane et vinylméthyloxy phénylsilane.

De tels polymères sont notamment décrits dans les documents US 3 175 993, US 4 772 675, US 4 871 827, US 4 888 380, US 4 898 910, US 4 906 719 et US 4 962 174 dont le contenu est incorporé par référence à la présente demande.

On peut citer à titre de composé X et/ou Y en particulier le polymère de formule

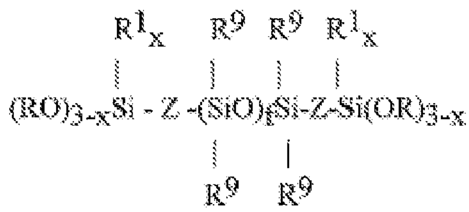

(VII)

dans laquelle R, $R^1$, $R^9$, Z, x et f sont tels que décrits plus haut.

Les composés X et/ou Y peuvent également comprendre un mélanges de polymère de formule (VII) ci-dessus avec des polymères de formule (VIII) suivante :

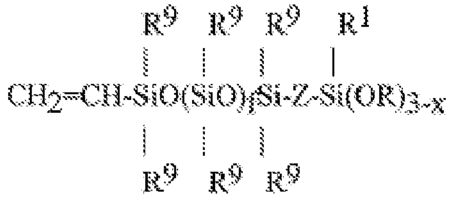

(VIII)

dans laquelle R, $R^1$, $R^9$, Z, x et f sont tels que décrits plus haut.

Lorsque le composé X et/ou Y polyorganosiloxanes à groupe(s) alcoxysilane(s) comprend un tel mélange, les différents polyorganosiloxanes sont présents en des teneurs telles que les chaînes organosilyles terminales représentent moins de 40%, de préférence moins de 25% en nombre des chaînes terminales

Les composés X et/ou Y polyorganosiloxanes particulièrement préférés sont ceux de formule (VII) décrits ci-dessus. De tels composés X et/ou Y sont décrits par exemple dans le document WO 01/96450.

Comme indiqué plus haut, les composés X et Y peuvent être identiques ou différents.

Selon une variante l'un des deux composés réactifs X ou Y, est de nature silicone et l'autre est de nature organique. Par exemple le composé X est choisi parmi les oligomères ou polymères organiques ou les oligomères ou polymères hybrides organique/silicone, lesdits polymères ou oligomères comprenant au moins deux groupements alcoxysilanes et Y est choisi parmi les composés siliconés tels que les polyorganosiloxanes décrits ci-dessus. En particulier, les oligomères ou polymères organiques sont choisis parmi les oligomères ou polymères vinyliques, (méth)acryliques, polyesters, polyamides, polyuréthanes et/ou polyurées, polyéthers, polyoléfines, perfluoropolyéthers, dendrimères et polymères hyper-ramifiés organiques, et leurs mélanges.

Les polymères organiques de nature vinylique ou (méth)acryliques, porteurs de groupes latéraux alcoxysilanes, pourront en particulier être obtenus par copolymérisation d'au moins un monomère organique vinylique ou (méth)acrylique avec un (méth)acryloxypropyltriméthoxysilane, un vinyl triméthoxysilane, un vinyltriéthoxysilane, un allyltriméthoxysilanes etc.

On peut citer par exemple les polymère (méth)acryliques décrits dans le document de KUSABE.M, Pitture e Verniei - European Coating ; 12-B, pages 43-49, 2005, et notamment les polyacrylates à groupes alcoxysilanes référencés MAX de Kaneka ou ceux décrits dans la publication de PROBSTER, M, Adhesion-Kleben & Dichten, 2004, 481 (1-2), pages 12-14.

Les polymères organiques résultant d'une polycondensation ou d'une polyaddition, tel que les polyesters, polyamides, polyuréthanes et/ou polyurées, polyéthers, et porteurs de groupes alcoysilanes latéraux et/ou terminaux, pourront résulter par exemple de la réaction d'un prépolymère oligomère tel que décrit plus haut avec l'un des co-réactifs silanes suivant porteurs d'au moins un groupe alcoxysilane : aminopropyltriméthoxysilane, aminopropyltriéthoxysilane, aminoéthyl aminopropyl triméthoxysilane, glycidoxypropyltriméthoxysilane, glycidoxypropyltriéthoxysilane, époxycyclohéxyléthyltriméthoxysilane, mercaptopropyltriméthoxysilane.

Des exemples de polyéthers et de polyisobutylènes à groupes alcoxysilanes sont décrits dans la publication de KUSABE.M., Pitture e Verniei - European Coating ; 12-B, pages 43-49, 2005. Comme exemple de polyuréthanes à groupes alcoxysilanes terminaux , on peut citer ceux décrits dans le document PROBSTER, M., Adhesion-Kleben & Dichten, 2004, 481 (1-2), pages 12-14 ou encore ceux décrits dans le document LANDON, S., Pitture e Verniei vol. 73, N° 11 , pages 18-24, 1997 ou dans le document HUANG, Mowo, Pitture e Verniei vol. 5, 2000, pages 61-67, on peut notamment citer les polyuréthanes à groupes alcoxysilanes de OSI-WITCO-GE.

A titre de composés X et/ou Y polyorganosiloxane, on peut citer les résines de type MQ ou MT portant ellemême des extrémités alcoxysilanes et/ou silanols comme par exemple les résines poly(isobutylsilsesquioxane) fonctionnalisées par des groupes silanols proposées sous la référence SST-S7C41 (3 groupes Si-OH) par la société Gelest.

## 2a Composé réactif additionnel

L'une des compositions utiles dans la présente invention peut comprendre en outre un composé réactif additionnel comprenant au moins deux groupes alcoxysilane ou silanol.

On peut citer par exemple une ou des particules organiques ou minérales comprenant à leur surface des groupements alcoxysilanes et/ou silanols, par exemple des charges traitées en surface par de tels groupes.

## 2b Catalyseur

La réaction de condensation peut se faire en présence d'un catalyseur à base de métal qui peut être présent

dans l'une ou l'autre des compositions comprenant X et/ou Y ou dans une composition séparée. Le catalyseur utile dans ce type de réaction est de préférence un catalyseur à base de titane.

On peut citer notamment les catalyseurs à base de tetraalcoxytitane de formule

$$Ti(OR^2)_y(OR^3)_{4-y},$$

dans laquelle $R^2$ est choisi parmi les radicaux alkyle tertiaires tels que le tert butyle, le tert amyle et le 2,4-diméthyl-3-pentyl ; $R^3$ représente un radical alkyle comprenant de 1 à 6 atomes de carbone, de préférence un groupe methyle ethyle, n-propyle, isopropyle, n-butyle, sec-butyle, hexyle et y est un nombre allant de 3 à 4, mieux de 3,4 à 4.

Le catalyseur peut être présent dans l'une ou l'autre des compositions utiles dans la présente invention en une teneur allant 0,0001% à 20% en poids par rapport au poids total de la ou des compositions le contenant.

**2c Diluant**

Les compositions utiles comprenant X et/ou Y peuvent comprendre en outre une huile siliconée volatile (ou diluant) destinée à faire diminuer la viscosité de la composition.

Cette huile peut être choisie parmi les silicones linéaires à chaîne courte telles que l'hexaméthyldisiloxane, l'octaméthyltrisiloxane, les silicones cycliques telles que l'octaméthylcyclotétrasiloxane, la décaméthylclopentasiloxane et leurs mélanges.

Cette huile siliconée peut représenter de 5% à 95%, de préférence de 10 à 80% en poids par rapport au poids de chaque composition.

A titre d'exemple d'une combinaison de composés X et Y porteurs de groupements alcoxysilanes et réagissant par condensation, on peut citer la combinaison des mélanges A' et B' suivants préparés par la société Dow Corning :

**Mélange A' :**

| Ingrédient (Nom INCI) | N°CAS | Teneurs (pds%) | Fonction |
|---|---|---|---|
| Bis-Trimethoxysiloxyethyl Tetramethyldisiloxyethyl Dimethicone | PMN87176 | 25-45 | Polymère |
| Silica Silylate | 68909-20-6 | 5-20 | Charge |
| Disiloxane | 107-46-0 | 30-70 | Solvant |

**Mélange B' :**

| Ingrédient (Nom INCI) | N°CAS | Teneurs (pds%) | Fonction |
|---|---|---|---|
| Disiloxane | 107-46-0 | 80-99 | Solvant |
| Tetra T Butyl Titanate | - | 1-20 | Catalyseur |

Il est par ailleurs à noter que les composés X et Y identiques sont réunis dans le mélange A'.

**3/ Réticulation en présence de peroxyde**

Cette réaction se fait de préférence par chauffage à une température supérieure ou égale à 50°C, de préférence supérieure ou égale à 80°C, allant jusqu'à 120°C.

Les composés X et Y, identiques ou différents, comprennent dans ce cas au moins deux groupements latéraux $-CH_3$ et/ou au moins deux chaînes latérales portant un groupement $-CH_3$.

Les composés X et Y sont de préférence siliconés et peuvent être choisis par exemple parmi les polydiméthylsiloxanes linéaires non volatiles de haut poids moléculaire, ayant un degré de polymérisation supérieur à 6 présentant au moins deux groupements latéraux $-CH_3$ reliés à l'atome de silicium et/ou au moins deux chaînes latérales portant

un groupement $-CH_3$. On peut citer par exemple les polymères décrits dans le Catalogue « Reactive Silicones » de la société Gelest Inc., Edition 2004, page 6, et notamment les copolymères (aussi appelés gommes) de vinylméthylsiloxane-diméthylsiloxane de poids moléculaires allant de 500 000 à 900 000 et notamment de viscosité supérieure à 2 000 000 cSt.

A titre de peroxydes utilisables dans le cadre de l'invention, on peut citer le peroxyde de benzoyle, le peroxyde de 2,4-dichlorobenzoyle et leurs mélanges.

Selon un mode de réalisation, la réaction d'hydrosilylation ou la réaction de condensation ou bien encore la réaction de réticulation en présence d'un peroxyde entre les composés X et Y est accélérée par un apport de chaleur en élevant par exemple la température du système entre 25°C et 180°C. Le système réagira notamment sur la peau.

D'une façon générale, quel que soit le type de réaction par laquelle les composés X et Y réagissent ensemble, le pourcentage molaire de X par rapport à l'ensemble des composés X et Y, c'est-à-dire le ratio X/(X+Y) x 100, peut varier de 5% à 95%, de préférence de 10% à 90%, mieux encore de 20% à 80%.

De même, le pourcentage molaire de Y par rapport à l'ensemble des composés X et Y, c'est-à-dire le ratio Y/(X+Y) x 100, peut varier de 5% à 95%, de préférence de 10% à 90%, mieux encore de 20% à 80%.

Le composé X peut présenter une masse moléculaire moyenne en poids (Mw) allant de 150 à 1 000 000, de préférence de 200 à 800 000, de préférence encore de 200 à 250 000.

Le composé Y peut présenter une masse moléculaire moyenne en poids (Mw) allant de 200 à 1 000 000, de préférence de 300 à 800 000, de préférence encore de 500 à 250 000.

Le composé X peut représenter de 5 % à 80% en poids par rapport au poids total de la composition, de préférence de 10 % à 50 % en poids.

Le composé Y peut représenter de 0,5 % à 80 % en poids par rapport au poids total de la composition, de préférence de 1 % à 50 % en poids.

Le ratio entre les composés X et Y peut être varié de manière à moduler la vitesse de réaction et donc la vitesse de formation du film ou encore de manière à adapter les propriétés du film formé (par exemple ses propriétés adhésives) selon l'application recherchée.

En particulier, si X et Y ne sont pas les mêmes, les composés X et Y peuvent être présents en un ratio X/Y molaire allant de 0,05 à 20 et mieux de 0,1 à 10.

## Les agents texturisants

La composition appliquée sur les fibres capillaires peut en outre comprendre un ou plusieurs agents texturisants (charges).

On entend par agents texturisants, des particules minérales ou de synthèse, lamellaires ou non lamellaires, insolubles dans l'eau.

A titre d'exemple, ces agents peuvent être du carbonate de calcium colloïdal qui peut être traité ou non par de l'acide stéarique ou du stéarate, de la silice telle que les silices pyrogénées, les silices précipitées, les silices traitées pour les rendre hydrophobes, du quartz moulu, de l'alumine, de l'hydroxyde d'aluminium, de la terre de diatomée.

On peut aussi citer le talc, le mica, le kaolin, les poudres de polyamide (Nylon@) (Orgasol de chez Atochem), les poudres de polyéthylène, les poudres de polymères de tétrafluoroéthylène (TéflonB), l'amidon, le nitrure de bore, des microsphères polymériques telles que celles de chlorure de polyvinylidène/acrylonitrile comme l'Expancel (Nobel Industrie), de copolymères d'acide acrylique (PolytrapB de la société Dow Corning).

Les silices synthétiques dont la surface est modifiée par des composés siliconés pour les rendre hydrophobe en surface sont particulièrement préférées. Ces charges se différentient les unes des autres par leurs propriétés de surface, les composés de silicone utilisés pour traiter la silice, et la façon dont le traitement en surface est réalisé.

De telles agents permettent de moduler la viscosité de la formulation obtenue à partir des composés X et/ou Y.

On préfère à titre de charge la silice, le carbonate de calcium, les charges à base de résine.

A titre d'exemple, on peut citer les charges traitées Cab-O-Sil@TS-530, Aerosil@R8200 et Wacker HDX H2000.

Elles peuvent être présentes à raison de 0 à 48 % en poids, par rapport au poids total de la composition, de préférence 0,01 à 30 % en poids, et mieux de 0,02 % à 20 % en poids.

## Additifs

La composition peut en outre comprendre au moins un additif cosmétique.

A titre d'exemple, on peut citer notamment les pigments classiques, les pigments à effet (par exemple les pigments fluorescents, photochromes ou thermochromes, les nacres ou les paillettes), les agents antipelliculaires ou anti-séborrhéiques, les parfums, les hydroxyacides, les électrolytes, les conservateurs, les filtres solaires siliconés ou non, les vitamines, les provitamines tels que le panthénol, les polymères anioniques ou non ioniques, les protéines, les hydrolysats de protéines, l'acide méthyl-18 eicosanoique, les huiles de synthèses telles que les poly-oléfines, les huiles minérales, les huiles végétales, les huiles fluorées ou perfluorées, les cires naturelles ou synthétiques, les composés

de type céramide, les esters d'acides carboxyliques, les silicones, les agents antioxydants, les agents séquestrants, les agents dispersants, des agents de conditionnement tels par exemple des cations, des silicones volatiles ou non volatiles, modifiées ou non modifiées différentes des composés X et Y, des agents filmogènes, des céramides, des agents conservateurs, des agents stabilisants, des agents opacifiants, ainsi que les mélanges de ces différents.

Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.

**Solvants organiques**

La composition mise en oeuvre dans le cadre de l'invention peut comprendre au moins un solvant organique.

Par solvant organique, on entend une substance organique liquide à la température de 25 °C et à pression atmosphérique (760 mm de Hg) capable de dissoudre une autre substance sans la modifier chimiquement.

Il est à noter que le ou les solvants organiques sont distincts des composés X et Y utiles dans le cadre de l'invention.

Le ou les solvants organiques sont par exemple choisis parmi les alcools aromatiques tels que l'alcool benzylique, le phénoxyéthanol, l'alcool phényléthylique ; les alcools gras liquides, notamment en $C_{10}$-$C_{30}$ les alcanols en $C_1$-$C_6$ comprenant une ou deux fonctions OH libres tels que l'éthanol, l'isopropanol, le n-propanol, le butanol, le n-pentanol, le 1,2-propanediol, le 1,3-propanediol, le 1-méthoxy 2-propanol, le 1-éthoxy 2-propanediol, les 1,3 et 1,4-butanediol, le 1,2-hexanediol ; les polyols et éthers de polyols possédant une fonction -OH libre tels que le 2-butoxyéthanol, le propylèneglycol, le monométhyléther de propylèneglycol, le monoéthyléther et le monométhyléther du diéthylèneglycol, l'éthylène glycol monoéthyléther, l'éthylène glycol mono butyl éther, le néopentyl glycol, l'isoprèneglycol, le glycérol, le glycol, le dipropylène glycol, le butylène glycol, le butyle diglycol ; les silicones volatiles telles que les silicones linéaires à chaîne courte telles que l'hexaméthyldisiloxane et l'octaméthyltrisiloxane, les silicones cycliques telles que l'octaméthylcyclotétrasiloxane, la décaméthylcyclopentasiloxane, la dodécaméthyl-cyclohexasiloxane, les polydiméthylsiloxanes modifiées ou non par des fonctions alkyle et/ou amine et/ou imine et/ou fluoroalkyl et/ou carboxylique et/ou betaïne et/ou ammonium quaternaire ; les polydiméthylsiloxanes modifiées liquides ; les huiles minérales, organiques ou végétales ; les alcanes et plus particulièrement les alcanes linéaires ou ramifiés de $C_5$ à $C_{20}$ ; les acides gras liquides ; les esters gras liquides et plus particulièrement les benzoates ou les salicylates d'alcool gras liquides.

Le ou les solvants organiques sont de préférence choisis parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ou encore des composés organiques tels que des alcanes tels que des alcanes linéaires ou ramifiés, en $C_5$-$C_{20}$ comme l'isododécane, l'isohexadécane, les composés isoparaffiniques du type des produits commercialisés sous la dénomination Isopar E, l'acétone, la méthyléthylcétone, les esters d'acides en $C_1$-$C_{20}$ liquides et d'alcools en $C_1$-$C_8$ tels que l'acétate de méthyle, l'acétate de butyle, l'acétate d'éthyle et le myristate d'isopropyle, le diméthoxyéthane, le diéthoxyéthane, les alcools gras liquides en $C_{10}$-$C_{30}$ tels que l'alcool oléique, les esters d'alcools gras ou d'acides gras liquides tels que les benzoates d'alcool gras en $C_{10}$-$C_{30}$ et leurs mélanges ; l'isononanoate d'isononyle, le malate d'isostéaryle, le tétra-isostéarate de pentaérythrityle, le trimélate de tridécyle ; l'huile de polybutène ; le mélange cyclopentasiloxane (14,7 % en poids) / polydiméthylsiloxane dihydroxylé en positions $\alpha$ et $\omega$ (85,3 % en poids), ou leurs mélanges.

Selon un mode de réalisation préféré, le ou les solvants organiques sont choisis parmi les silicones telles que les polydiméthylsiloxanes liquides et les polydiméthylsiloxanes modifiées liquides, leur viscosité à 25 °C étant comprise entre 0,1 cst et 1 000 000 cst, et plus préférentiellement entre 1 cst et 30 000 cst.

On citera de préférence les huiles suivantes :

- le mélange de polydiméthylsiloxane alpha-omega-dihydroxylé / cyclopentadiméthylsiloxane (14,7 / 85,3) commercialisé par Dow Corning sous le nom de DC 1501 Fluid ;
- le mélange de polydiméthylsiloxane alpha-omega-dihydroxylé / polydiméthylsiloxane commercialisé par Dow Corning sous le nom de DC 1503 Fluid ;
- le mélange de diméthicone / cyclopentadiméthylsiloxane commercialisé par Dow Corning sous le nom de DC 1411 Fluid ou celui commercialisé par Bayer sous le nom SF1214 ;
- la cyclopentadiméthylsiloxane commercialisée par Dow Corning sous le nom de DC245 Fluid ;
- et les mélanges respectifs de ces huiles.

Le ou les solvants organiques de la composition représentent de préférence de 20 à 85 % en poids par rapport au poids total de la composition.

De préférence, la composition de l'invention est anhydre, c'est-à-dire qu'elle comprend moins de 1 % en poids

d'eau par rapport au poids total de la composition.

Ces compositions peuvent se présenter sous des formes diverses, telles que des lotions, des aérosols, des mousses, des émulsions et être appliquées sous forme de shampooing ou d'après-shampooing.

Dans le cas des aérosols, la composition de l'invention peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

On pourra aussi utiliser des aérosols à poche(s) contenant une ou plusieurs poches. Pour l'application des produits de manière générale, on pourra utiliser tout dispositif comportant des zones de stockage multiples et un système de délivrance avec un ou plusieurs orifices permettant le mélange des produits à la sortie de ce dispositif.

La composition selon l'invention peut être appliquée sur cheveux secs ou humides.

La composition comprenant le ou les composés X, Y, peut être obtenue par mélange extemporané de plusieurs compositions comprenant un ou plusieurs des ingrédients. Ainsi, on peut envisager mélanger une composition comprenant au moins le composé X avec une composition comprenant au moins le composé Y. Au cas où un catalyseur ou un peroxyde serait présent, ce dernier peut se trouver dans l'une et/ou l'autre des compositions précitées ou dans une composition séparée.

L'invention est illustrée plus en détails par les exemples décrits ci-après.

Sauf indication contraire, les quantités indiquées sont exprimées en pourcentage massique.

**Exemple 1**

Dans cet exemple, on utilise les mélanges A' et B' suivants préparés par Dow Corning :

**Mélange A' :**

| Ingrédient (Nom INCI) | N°CAS | Teneurs (pds%) | Fonction |
|---|---|---|---|
| Bis-Trimethoxysiloxyethyl Tetramethyldisiloxyethyl Dimethicone | PMN87176 | 25-45 | Polymère |
| Silica Silylate | 68909-20-6 | 5-20 | Charge |
| Disiloxane | 107-46-0 | 30-70 | Solvant |

**Mélange B' :**

| Ingrédient (Nom INCI) | N°CAS | Teneurs (pds%) | Fonction |
|---|---|---|---|
| Disiloxane | 107-46-0 | 80-99 | Solvant |
| Tetra T Butyl Titanate | - | 1-20 | Catalyseur |

On prépare les compositions suivantes :

<u>Composition 1</u>

| | |
|---|---|
| Mélange A' | 99,5 % |
| Prestige Dazzling Red Gold (Eckart) | 0,5 |

Composition 2

| | |
|---|---|
| Mélange B' | 100% |

Les compositions 1 et 2 sont mélangées avec le rapport pondéral 10/1 avant l'application sur cheveux humides.
Les cheveux sont séchés à l'air libre pendant 30 minutes puis mis sous casque pendant 30 minutes.
Nous obtenons des coiffures méchées à effet paillettes, résistant à plusieurs shampooings.

**Exemple 2**

Dans cet exemple, on utilise les mélanges A et B suivants, préparés par DowCorning :

**Mélange A :**

| Ingrédient (Nom INCI) | N°CAS | Teneurs (pds%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

**Mélange B :**

| Ingrédient (Nom INCI) | N°CAS | Teneurs (pds%) | Fonction |
|---|---|---|---|
| Dimethyl Siloxane, Dimethylvinylsiloxy-terminated | 68083-19-2 | 55-95 | Polymère |
| Silica Silylate | 68909-20-6 | 10-40 | Charge |
| Dimethyl, Methylhydrogen Siloxane, trimethylsiloxy-terminated | 68037-59-2 | 1-10 | Polymère |

On prépare les compositions suivantes :

Composition 1

| | |
|---|---|
| Mélange A | 50 % |
| Dow Corning 5225C Formulation Aid | 10 % |
| Cyclopentadiméthylsiloxane commercialisé par Dow Corning sous le nom de DC245 Fluid | 39,8 % |
| Prestige Dazzling Red Gold (Eckart) | 0,2 % |

Composition 2

| | |
|---|---|
| Mélange B | 50 % |
| Cyclopentadiméthylsiloxane commercialisé par Dow Corning sous le nom de DC245 Fluid | 50 % |

Les compositions 1 et 2 sont mélangées avec le rapport pondéral 1/1 avant l'application sur cheveux secs ou humides.
Les cheveux sont séchés à l'air libre pendant 30 minutes puis mis sous casque pendant 30 minutes.
Nous obtenons des coiffures méchées à effet paillettes, résistant à plusieurs shampooings.

**Exemple 3**

Dans cet exemple, on utilise les mélanges A' et B' de l'exemple 1.
On prépare deux dispositifs de spray aérosol avec les compositions suivantes :

| **Aérosol 1 : Composition 1** | en g |
|---|---|
| Cyclopentadiméthylsiloxane commercialisé par Dow Corning sous le nom de DC245 Fluid | 45 |
| Mélange A' | 15 |
| Diméthyléther | 40 |

| **Aérosol 2 : Composition 2** | en g |
|---|---|
| Cyclopentadiméthylsiloxane commercialisé par Dow Corning sous le nom de DC245 Fluid | 58,5 |
| Mélange B' | 1,5 |
| Diméthyléther | 40 |

On construit une coiffure méchée avec l'aérosol 1 puis on applique l'aérosol 2 en finition.
La coiffure a une très bonne tenue.
Les méchés résistent à plusieurs shampooings.

## Revendications

1. Procédé de traitement de fibres kératiniques humaines, telles que les cheveux, pour l'obtention de coiffures structurées, dans lequel on applique une composition comprenant au moins 5 % en poids d'un composé X, au moins 0,5 % en poids d'un composé Y, au moins un des composés X ou Y étant siliconé, lesdits composés X et Y étant susceptibles de réagir ensemble par une réaction d'hydrosilylation, ou par une réaction de condensation ou par une réaction de réticulation en présence d'un peroxyde.

2. Procédé selon la revendication 1, **caractérisé en ce que** la coiffure structurée est une coiffure permettant d'obtenir des mèches comprenant un ensemble d'au moins 10 fibres, de préférence d'au moins 50 fibres, collées entre elles.

3. Procédé selon la revendication 1, **caractérisé en ce que** l'on applique simultanément ou séparément au moins un catalyseur.

4. Procédé selon l'une des revendications précédentes, **caractérisé en ce que** les composés X et Y sont susceptibles de réagir par hydrosilylation.

5. Procédé selon la revendication 3, **caractérisé en ce que** le composé X est choisi parmi les composés siliconés comprenant au moins deux groupements aliphatiques insaturés.

6. Procédé selon la revendication 4, **caractérisé en ce que** le composé X est un polyorganosiloxane comprenant une chaîne principale siliconée dont les groupements aliphatiques insaturés sont pendants à la chaîne principale (groupe

latéral) ou situés aux extrémités de la chaîne principale du composé (groupe terminal).

**7.** Procédé selon l'une quelconque des revendications 3 à 5, **caractérisé en ce que** le composé X est choisi parmi les polyorganosiloxanes comprenant au moins deux groupements aliphatiques insaturés liés chacun à un atome de silicium

**8.** Procédé selon l'une des revendications 3 à 6, **caractérisé en ce que** le composé X est choisi parmi les polyorganosiloxanes comprenant des unités siloxanes de formule :

$$R_m R' SiO_{\frac{(3-m)}{2}} \quad (I)$$

dans laquelle :

- R représente un groupe hydrocarboné monovalent, linéaire ou cyclique, comprenant de 1 à 30 atomes de carbone,
- m est égal à 1 ou 2 et
- R' représente un groupement hydrocarboné aliphatique insaturé comprenant de 2 à 10, de préférence de 3 à 5 atomes de carbone ou un groupement hydrocarboné cyclique insaturé comprenant de 5 à 8 atomes de carbone.

**9.** Procédé selon la revendication 7, **caractérisé en ce que** le polyorganosiloxane de formule (I) est tel que R' représente un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène.

**10.** Procédé selon la revendication 7 ou 8, **caractérisé en ce que** R représente un radical alkyle comprenant de 1 à 10 atomes de carbone ou encore un groupement phényle et R' est un groupe vinyle.

**11.** Procédé selon l'une des revendications 3 à 9, **caractérisé en ce que** les polyorganosiloxanes comprennent en outre des unités de formule

$$R_n SiO_{\frac{(4-n)}{2}} \quad (II)$$

dans laquelle R représente un groupe hydrocarboné monovalent, linéaire ou cyclique, comprenant de 1 à 30 atomes de carbone, et n est égal à 1, 2 ou 3.

**12.** Procédé selon la revendication 3, **caractérisé en ce que** le composé X est choisi parmi les oligomères ou polymères organiques, les oligomères ou polymères hybrides organique/silicone, lesdits oligomères ou polymères portant au moins 2 groupements aliphatiques insaturés réactifs et leurs mélanges.

**13.** Procédé selon l'une des revendications 3 à 11, **caractérisé en ce que** le composé Y est choisi parmi les organosiloxanes comprenant au moins deux groupes Si-H libres.

**14.** Procédé selon l'une des revendications 3 à 12, **caractérisé en ce que** le composé Y est choisi parmi les organosiloxanes comprenant au moins une unité alkylhydrogènosiloxanes de formule suivante :

$$R_pHSiO_{\left(\frac{3-p}{2}\right)} \quad (III)$$

dans laquelle :

R représente un groupe hydrocarboné monovalent, linéaire ou cyclique, comprenant de 1 à 30 atomes de carbone ou un groupe phényle, et p est égal à 1 ou 2.

**15.** Procédé selon la revendication précédente, **caractérisé en ce que** le composé Y est tel que les radicaux R représentent un groupement alkyle en $C_1$-$C_{10}$, de préférence méthyle.

**16.** Procédé selon l'une quelconque des revendications 12 à 14, **caractérisé en ce que** les organosiloxanes Y comprennent au moins deux unités alkylhydrogénosiloxane de formule $-(H_3C)(H)Si-O-$ et comprennent éventuellement des unités $-(H_3C)_2SiO-$

**17.** Procédé selon l'une des revendications 3 à 15, **caractérisé en ce que** le catalyseur est un catalyseur à base de platine ou d'étain présent dans l'une ou l'autre des compositions comprenant X et/ou Y ou dans une composition séparée.

**18.** Procédé selon la revendication précédente, **caractérisé en ce que** le catalyseur représente de 0,0001 % à 20% en poids par rapport au poids total de la composition.

**19.** Procédé selon l'une des quelconque des revendications 3 à 12, **caractérisé en ce que** le composé X est un polydiméthylsiloxane à groupements vinyliques terminaux et le composé Y est un méthylhydrogénosiloxane.

**20.** Procédé selon l'une des revendications 1 ou 2, **caractérisé en ce que** le composé X et le composé Y sont susceptibles de réagir par condensation.

**21.** Procédé selon la revendication 19, **caractérisé en ce que** les composés X et/ou Y, identiques ou différents sont des composés siliconés dont la chaîne principale comprend au moins deux groupes alcoxysilane et/ou au moins deux groupes silanol (Si-OH), latéraux et/ou en bout de chaîne.

**22.** Procédé selon la revendication 19 ou 20, **caractérisé en ce que** les composés X et/ou Y, identiques ou différents comprennent de façon majoritaire des unités de formule

$$R^9{}_8SiO_{(4-s)/2}, \quad (IV)$$

dans laquelle les $R^9$ représentent indépendamment un radical choisi parmi les groupes alkyles comprenant de 1 à 6 atomes de carbone, le phényle, les groupes alkyl fluorés, et s est égal à 0, 1, 2 ou 3.

**23.** Procédé selon la revendication 21, **caractérisé en ce que** les composés X et/ou Y, identiques ou différents comprennent des unités de formule

$$(R^9{}_2SiO_2)_f- \quad (V)$$

dans laquelle $R^9$ est tel que défini dans la revendication 21, et f est tel que le polymère présente avantageusement une viscosité à 25°C allant de 0,5 à 3000 Pa.s, et/ou f est un nombre allant de 2 à 5000.

**24.** Procédé selon l'une des revendications 19 à 22, **caractérisé en ce que** les polyorganosiloxanes comprennent au moins deux groupes trialcoxysilane terminaux par molécule de polymère, lesdits groupes ayant la formule suivante

$$-ZSiR^1x(OR)_{3-x}, \quad (VI)$$

dans laquelle
les radicaux R représentent indépendamment un groupe méthyle, éthyle, n-propyle, isopropyle, n-butyle, sec-butyle,

isobutyle,
$R^1$ est un groupe méthyle ou éthyle,
x est égal à 0 ou 1, de préférence x est égal à 0 et
Z est choisi parmi : les groupes hydrocarbonés divalents ne comportant pas d'insaturation éthylénique et comprenant de 2 à 18 atomes de carbone, les combinaisons de radicaux hydrocarbonés divalents et de segments siloxanes de formule (IX) :

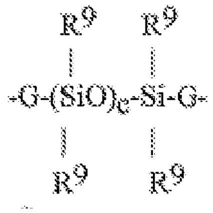

$R^9$ étant tel que défini dans la revendication 22, G est un radical hydrocarboné divalent ne comportant pas d'insaturation éthylénique et comprenant de 2 à 18 atomes de carbone et c est un entier allant de 1 à 6.

**25.** Procédé selon l'une des revendications 19 à 23, **caractérisé en ce que** les polyorganosiloxanes sont choisis parmi les polymères de formule :

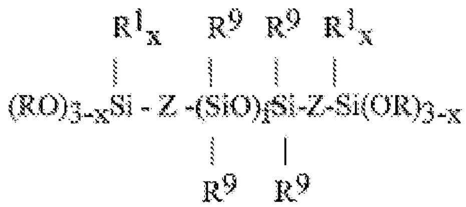

(VII)

dans laquelle R, $R^1$, $R^9$, Z, x et f sont tels que définis dans l'une des revendications 21 à 23.

**26.** Procédé selon la revendication 19, **caractérisé en ce que** le composé X est choisi parmi les oligomères ou polymères organiques ou les oligomères et polymères hybrides organique/silicone, lesdits polymères ou oligomères comprenant au moins deux groupements alcoxysilanes et le composé Y est choisi parmi les polyorganosiloxanes.

**27.** Procédé selon l'une des revendications 19 à 25, **caractérisé en ce que** au moins l'une des compositions comprend un catalyseur à base de titane.

**28.** Procédé selon la revendication 26, **caractérisé en ce que** le catalyseur est présent en une teneur allant de 0,0001% à 20% en poids par rapport au poids total de la composition.

**29.** Procédé selon l'une quelconque des revendications 19 à 27, **caractérisé en ce que** les composés X et Y représentent un mélange de polydiméthylsiloxanes à groupements méthoxysilanes.

**30.** Procédé selon la revendication 1 ou 2, **caractérisé en ce que** les composés X et Y sont susceptibles de réagir par réticulation en présence de peroxyde.

**31.** Procédé selon l'une quelconque des revendications précédentes, **caractérisé en ce que** la composition comprend au moins un agent texturisant.

**32.** Procédé selon l'une quelconque des revendications précédentes, **caractérisé en ce que** la composition comprend au moins un pigment.

**33.** Procédé selon la revendication précédente, **caractérisé en ce que** la composition comprend au moins un pigment à effet.

**34.** Procédé selon l'une des revendications précédentes, **caractérisé en ce que** le composé X présente une masse moléculaire moyenne en poids (Mw) allant de 150 à 1 000 000, de préférence de 200 à 800 000, de préférence encore de 200 à 250 000.

**35.** Procédé selon l'une des revendications précédentes, **caractérisé en ce que** le composé Y présente une masse moléculaire en poids (Mw) allant de 200 à 1 000 000, de préférence de 300 à 800 000, de préférence encore de 500 à 250 000.

**36.** Procédé selon l'une des revendications précédentes, **caractérisé en ce que** le composé X représente de 5 % à 80 % en poids par rapport au poids total de la composition, de préférence de 10 % à 50 % en poids.

**37.** Procédé selon l'une des revendications précédentes, **caractérisé en ce que** le composé Y représente de 0,5 % à 80 % en poids par rapport au poids total de la composition, de préférence de 1 % à 50 % en poids.

**38.** Procédé selon l'une des revendications précédentes, **caractérisé en ce que** les composés X et Y sont présents dans les compositions en un ratio molaire X/Y allant de 0,05 à 20 et mieux de 0,1 à 10.

**39.** Procédé selon l'une quelconque des revendications précédentes, **caractérisé en ce que** la composition comprend au moins un solvant organique.

**40.** Procédé selon l'une quelconque des revendications précédentes, **caractérisé en ce que** le solvant organique est choisi parmi les alcools aromatiques ; les alcools gras liquides, notamment en $C_{10}$-$C_{30}$ les alcanols en $C_1$-$C_6$ comprenant une ou deux fonctions OH libres ; les polyols et éthers de polyols possédant une fonction OH libre ; les silicones volatiles ; les polydiméthylsiloxanes modifiées liquides ; les huiles minérales, organiques ou végétales ; les alcanes et plus particulièrement les alcanes de $C_5$ à $C_{20}$ ; les acides gras liquides ; les esters gras liquides, ou leurs mélanges.

**41.** Procédé selon l'une quelconque des revendications précédentes, **caractérisé en ce que** la quantité de solvant est comprise entre 20 et 85 % en poids par rapport au poids total de la composition.

Office européen des brevets

# RAPPORT DE RECHERCHE EUROPEENNE

Numéro de la demande
EP 07 12 3272

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | WO 2006/028612 A (DOW CORNING [US]; MURPHY KEVIN PATRICK [US]; SCHALAU GERALD KENNETH II) 16 mars 2006 (2006-03-16)<br>* alinéas [0008], [0012], [0013], [0074], [0075] * | 1-19, 21-29, 31-41 | INV.<br>A61Q5/06<br>A61K8/895<br>A61K8/89 |
| X | US 5 811 085 A (HALLORAN DANIEL J [US]) 22 septembre 1998 (1998-09-22)<br>* revendications *<br>* colonne 4, ligne 43 - ligne 48 *<br>* colonne 5, ligne 25 - ligne 43 *<br>* colonne 6, ligne 20 - ligne 21 *<br>* exemples * | 1-3,5, 7-19, 21-41 | |
| X | WO 2004/084847 A (UNILEVER PLC [GB]; UNILEVER NV [NL]; LEVER HINDUSTAN LTD [IN]; IVANOVA) 7 octobre 2004 (2004-10-07)<br>* page 2, ligne 1 - ligne 31 *<br>* page 4, ligne 10, alinéa I - page 10, ligne 2 *<br>* page 34, ligne 1 - ligne 4 *<br>* exemples 1-3,b,c * | 1-41 | **DOMAINES TECHNIQUES RECHERCHES (IPC)** |
| | | | A61K<br>A61Q |
| A | DE 200 05 808 U1 (WELLA AG) 20 juillet 2000 (2000-07-20)<br>* page 15, ligne 4 - ligne 15 * | 1-19, 21-29, 31-41 | |
| A | EP 1 101 486 A1 (OREAL [FR]) 23 mai 2001 (2001-05-23)<br>* exemples * | 1-19, 21-29, 31-41 | |

-/--

Le présent rapport a été établi pour toutes les revendications

| Lieu de la recherche | Date d'achèvement de la recherche | Examinateur |
|---|---|---|
| Munich | 15 juillet 2008 | Krattinger, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

7

EPO FORM 1503 03.82 (P04C02)

Office européen des brevets

# RAPPORT DE RECHERCHE EUROPEENNE

Numéro de la demande
EP 07 12 3272

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | EP 1 190 699 A2 (WELLA AG [DE]) 27 mars 2002 (2002-03-27)<br>* alinéa [0002]; revendications; exemples * | 1-19, 21-29, 31-41 | |
| P,X | WO 2007/071886 A (OREAL [FR]; BENABDILLAH KATARINA [FR]; COTHIAS PASCALE [FR]) 28 juin 2007 (2007-06-28)<br>* exemples *<br>* revendications *<br>* page 2, ligne 23 - ligne 26 * | 1-41 | |
| E,L | EP 1 938 865 A (OREAL [FR]) 2 juillet 2008 (2008-07-02)<br>* alinéa [0009] * | 1-41 | |
| E | EP 1 941 930 A (OREAL [FR]) 9 juillet 2008 (2008-07-09)<br>* alinéas [0008], [0012], [0063] - [0084]; exemple 1 * | 29 | DOMAINES TECHNIQUES RECHERCHES (IPC) |
| D,X | US 4 344 763 A (TOLGYESI EVA ET AL) 17 août 1982 (1982-08-17)<br>* revendications; exemples * | 1-3,5, 7-41 | |
| X | EP 1 312 352 A (OREAL [FR]) 21 mai 2003 (2003-05-21)<br>* revendications 1-6,31; exemple 3 * | 1-3,5, 7-41 | |
| X | US 4 902 499 A (BOLISH JR RAYMOND E [US] ET AL) 20 février 1990 (1990-02-20)<br>* exemples 2-4,7-9 *<br>* revendications 1-5 *<br>* colonne 1, ligne 13 - ligne 16 *<br>* colonne 2, ligne 55 - ligne 59 * | 1-3,5, 7-19, 21-41 | |
| | -/-- | | |

Le présent rapport a été établi pour toutes les revendications

| Lieu de la recherche | Date d'achèvement de la recherche | Examinateur |
|---|---|---|
| Munich | 15 juillet 2008 | Krattinger, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

7

Office européen des brevets

# RAPPORT DE RECHERCHE EUROPEENNE

Numéro de la demande
EP 07 12 3272

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2 840 087 A (HERSH HERMAN I) 24 juin 1958 (1958-06-24)<br>* exemples 2,4 *<br>* colonne 1, ligne 15 - ligne 21 *<br>----- | 1-3,5, 7-41 | |
| X | US 2 787 274 A (GANT VIRGIL A ET AL) 2 avril 1957 (1957-04-02)<br>* exemples 2-5 *<br>* revendications *<br>* colonne 2, ligne 1 - ligne 10 *<br>----- | 1-3,5, 7-41 | |
| X | EP 0 159 628 A (REVLON [US]) 30 octobre 1985 (1985-10-30)<br>* exemples *<br>* revendications *<br>* page 1, ligne 1 - ligne 3 *<br>* page 3, ligne 9 - ligne 15 *<br>----- | 1-3,5, 7-41 | |
| X | EP 0 473 039 A (DOW CORNING [US]) 4 mars 1992 (1992-03-04)<br>* revendications 1-7 *<br>* page 3, ligne 11 - ligne 13 *<br>* page 4, ligne 13 - page 6, ligne 46 *<br>* exemples *<br>----- | 1-3,5, 7-41 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 15 juillet 2008 | Krattinger, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

7

EPO FORM 1503 03.82 (P04C02)

Office européen
des brevets

Numéro de la demande
EP 07 12 3272

## REVENDICATIONS DONNANT LIEU AU PAIEMENT DE TAXES

La présente demande de brevet européen comportait lors de son dépôt les revendications dont le paiement était dû.

☐ Une partie seulement des taxes de revendication ayant été acquittée dans les délais prescrits, le présent rapport de recherche européenne a été établi pour les revendications pour lesquelles aucun paiement n'était dû ainsi que pour celles dont les taxes de revendication ont été acquittées, à savoir les revendication(s):

☐ Aucune taxe de revendication n'ayant été acquittée dans les délais prescrits, le présent rapport de recherche européenne a été établi pour les revendications pour lesquelles aucun paiement n'était dû.

## ABSENCE D'UNITE D'INVENTION

La division de la recherche estime que la présente demande de brevet européen ne satisfait pas à l'exigence relative à l'unité d'invention et concerne plusieurs inventions ou pluralités d'inventions, à savoir:

voir feuille supplémentaire B

☒ Toutes les nouvelles taxes de recherche ayant été acquittées dans les délais impartis, le présent rapport de recherche européenne a été établi pour toutes les revendications.

☐ Comme toutes les recherches portant sur les revendications qui s'y prêtaient ont pu être effectuées sans effort particulier justifiant une taxe additionnelle, la division de la recherche n'a sollicité le paiement d'aucune taxe de cette nature.

☐ Une partie seulement des nouvelles taxes de recherche ayant été acquittée dans les délais impartis, le présent rapport de recherche européenne a été établi pour les parties qui se rapportent aux inventions pour lesquelles les taxes de recherche ont été acquittées, à savoir les revendications:

☐ Aucune nouvelle taxe de recherche n'ayant été acquittée dans les délais impartis, le présent rapport de recherche européenne a été établi pour les parties de la demande de brevet européen qui se rapportent à l'invention mentionnée en premier lieu dans les revendications, à savoir les revendications:

☐ Le present rapport supplémentaire de recherche européenne a été établi pour les parties de la demande de brevet européen qui se rapportent a l'invention mentionée en premier lieu dans le revendications (Règle 164 (1) CBE)

Office européen des brevets

# ABSENCE D'UNITÉ D'INVENTION FEUILLE SUPPLÉMENTAIRE B

Numéro de la demande

EP 07 12 3272

La division de la recherche estime que la présente demande de brevet européen ne satisfait pas à l'exigence relative à l'unité d'invention et concerne plusieurs inventions ou pluralités d'inventions, à savoir :

1. revendications: 1-3 (partie), 4, 5 (partie),6, 7-19 (partie), 21-29 (partie), 31-41 (partie)

   Procédés impliquant des ingrédients susceptibles de réagir par hydrosilylation

   ---

2. revendications: 1-3 (partie), 5 (partie), 7-19 (partie), 20, 21-29 (partie), 31-41 (partie)

   Procédés impliquant des ingrédients susceptibles de réagir par condensation

   ---

3. revendications: 1-3 (partie), 5 (partie), 7-19 (partie), 21-29 (partie), 30, 31-41 (partie)

   Procédés impliquant des ingrédients susceptibles de réagir par réticulation en présence d'un peroxyde

   ---

EPO FORM P0402

**ANNEXE AU RAPPORT DE RECHERCHE EUROPEENNE RELATIF A LA DEMANDE DE BREVET EUROPEEN NO.** EP 07 12 3272

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-07-2008

| Document brevet cité au rapport de recherche | Date de publication | Membre(s) de la famille de brevet(s) | Date de publication |
|---|---|---|---|
| WO 2006028612 A | 16-03-2006 | CN 101010065 A | 01-08-2007 |
| | | EP 1793811 A1 | 13-06-2007 |
| | | JP 2008512373 T | 24-04-2008 |
| | | KR 20070049653 A | 11-05-2007 |
| US 5811085 A | 22-09-1998 | CA 2033576 A1 | 10-07-1991 |
| | | DE 69005283 D1 | 27-01-1994 |
| | | EP 0437075 A2 | 17-07-1991 |
| | | JP 4210905 A | 03-08-1992 |
| WO 2004084847 A | 07-10-2004 | JP 2006521303 T | 21-09-2006 |
| | | US 2006280693 A1 | 14-12-2006 |
| DE 20005808 U1 | 20-07-2000 | BR 0002513 A | 02-01-2001 |
| | | EP 1055407 A2 | 29-11-2000 |
| | | JP 2000344637 A | 12-12-2000 |
| | | US 6368581 B1 | 09-04-2002 |
| EP 1101486 A1 | 23-05-2001 | AT 333925 T | 15-08-2006 |
| | | AU 747931 B2 | 30-05-2002 |
| | | AU 7148000 A | 24-05-2001 |
| | | BR 0005647 A | 19-06-2001 |
| | | CA 2325900 A1 | 19-05-2001 |
| | | CN 1300587 A | 27-06-2001 |
| | | DE 60029540 T2 | 12-07-2007 |
| | | ES 2267478 T3 | 16-03-2007 |
| | | FR 2801202 A1 | 25-05-2001 |
| | | JP 3669916 B2 | 13-07-2005 |
| | | JP 2001163722 A | 19-06-2001 |
| | | JP 2005139202 A | 02-06-2005 |
| | | KR 20010051731 A | 25-06-2001 |
| | | PL 343950 A1 | 21-05-2001 |
| | | RU 2200533 C2 | 20-03-2003 |
| | | US 6432386 B1 | 13-08-2002 |
| EP 1190699 A2 | 27-03-2002 | AT 346587 T | 15-12-2006 |
| | | DE 10047038 C1 | 05-09-2002 |
| | | ES 2276729 T3 | 01-07-2007 |
| | | US 2002076424 A1 | 20-06-2002 |
| WO 2007071886 A | 28-06-2007 | FR 2894817 A1 | 22-06-2007 |
| EP 1938865 A | 02-07-2008 | FR 2910312 A1 | 27-06-2008 |
| EP 1941930 A | 09-07-2008 | FR 2910311 A1 | 27-06-2008 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 07 12 3272

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-07-2008

| Document brevet cité au rapport de recherche | Date de publication | Membre(s) de la famille de brevet(s) | Date de publication |
|---|---|---|---|
| US 4344763 A | 17-08-1982 | CA 1112181 A1 | 10-11-1981 |
| EP 1312352 A | 21-05-2003 | AU 2002301888 B2 | 25-11-2004 |
| | | BR 0204979 A | 16-09-2003 |
| | | CA 2411104 A1 | 08-05-2003 |
| | | CN 1424016 A | 18-06-2003 |
| | | FR 2831818 A1 | 09-05-2003 |
| | | JP 3950991 B2 | 01-08-2007 |
| | | JP 2003146850 A | 21-05-2003 |
| | | JP 2006249107 A | 21-09-2006 |
| | | KR 20030038512 A | 16-05-2003 |
| | | MX PA02010941 A | 16-07-2004 |
| | | PL 357008 A1 | 19-05-2003 |
| | | RU 2229281 C1 | 27-05-2004 |
| | | US 2003152543 A1 | 14-08-2003 |
| | | ZA 200209049 A | 23-05-2003 |
| US 4902499 A | 20-02-1990 | AU 605144 B2 | 10-01-1991 |
| | | AU 7105787 A | 08-10-1987 |
| | | CA 1285492 C | 02-07-1991 |
| | | JP 7029906 B | 05-04-1995 |
| | | JP 63022010 A | 29-01-1988 |
| | | MX 166507 B | 13-01-1993 |
| US 2840087 A | 24-06-1958 | AUCUN | |
| US 2787274 A | 02-04-1957 | AUCUN | |
| EP 0159628 A | 30-10-1985 | AU 571671 B2 | 21-04-1988 |
| | | AU 4090685 A | 17-10-1985 |
| | | CA 1254144 A1 | 16-05-1989 |
| | | DE 3578680 D1 | 23-08-1990 |
| | | JP 61000007 A | 06-01-1986 |
| | | ZA 8502756 A | 24-12-1985 |
| EP 0473039 A | 04-03-1992 | CA 2046137 A1 | 21-02-1992 |
| | | DE 69105411 D1 | 12-01-1995 |
| | | DE 69105411 T2 | 04-05-1995 |
| | | JP 2874747 B2 | 24-03-1999 |
| | | JP 4261113 A | 17-09-1992 |
| | | US 5089253 A | 18-02-1992 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4344763 A
- FR 2833489
- EP 0465744 A
- EP 959066 A
- EP 1057849 A
- WO 9317060 A
- WO 9612754 A
- US 3175993 A
- US 4772675 A
- US 4871827 A
- US 4888380 A
- US 4898910 A
- US 4906719 A
- US 4962174 A
- WO 0196450 A


### Littérature non-brevet citée dans la description


- **DONALD A. TOMALIA et al.** *Angewandte Chemie, Int. Engl. Ed.,* vol. 29 (2), 138-175
- **KUSABE.M.** *Pitture e Verniei - European Coating,* 2005, vol. 12-B, 43-49
- **PROBSTER, M.** *Adhesion-Kleben & Dichten,* 2004, vol. 481 (1-2), 12-14
- **LANDON, S.** *Pitture e Verniei,* 1997, vol. 73 (11), 18-24
- **HUANG, MOWO.** *Pitture e Verniei,* 2000, vol. 5, 61-67
- Reactive Silicones. Gelest Inc, 2004, 6